# EUROPEAN PATENT SPECIFICATION

(11) **EP 2 268 201 B1**
(45) Date of publication and mention of the grant of the patent: **17.05.2017**
(21) Application number: 09732993.2
(22) Date of filing: 14.04.2009
(51) Int. Cl.: A61B 5/0275

(54) **EVALUATION OF PERFUSION OF PERFORATOR VESSELS**
BEWERTUNG DER PERFUSION VON PERFORANSGEFÄßEN
EVALUATION DE PERFUSION DE VAISSEAUX PERFORANTS

(30) Priority: 14.04.2008 US 44779 P
(43) Date of publication of application: 05.01.2011
(73) Proprietor: Novadaq Technologies Inc., Mississauga, ON L4W 4T9 (CA)
(72) Inventor: DVORSKY, Peter, Toronto Ontario M4L 3X5 (CA); GOYETTE, David M. H., Mississauga Ontario L5M 2J9 (CA); FERGUSON, Jr., T. Bruce, Raleigh, NC 27615 (US); CHEN, Cheng, Greenville, NC 27834 (US)
(74) Representative: V.O.
(86) International application number: PCT/IB2009/005700
(87) International publication number: WO 2009/127972

(56) References cited:
- EP-A2- 1 761 171
- WO-A1-2004/052195
- DE-A1- 3 906 860
- JP-A- 2008 023 113
- US-A1- 2002 183 621
- US-A1- 2005 182 434
- US-B1- 6 915 154
- US-B2- 6 757 554
- HOLM ET AL: "Intraoperative evaluation of skin-flap viability using laser-induced fluorescence of indocyanine green", BRITISH JOURNAL OF PLASTIC SURGERY, CHURCHILL LIVINGSTONE, GB, vol. 55, no. 8, 1 December 2002 (2002-12-01), pages 635-644, XP005315304, ISSN: 0007-1226, DOI: 10.1054/BJPS.2002.3969
- KRISHNAN K G ET AL: "The role of near-infrared angiography in the assessment of post-operative venous congestion in random pattern, pedicled island and free flaps", BRITISH JOURNAL OF PLASTIC SURGERY, CHURCHILL LIVINGSTONE, GB, vol. 58, no. 3, 1 April 2005 (2005-04-01), pages 330-338, XP004787587, ISSN: 0007-1226, DOI: 10.1016/J.BJPS.2004.10.003
- FRENZEL H ET AL: "In vivo perfusion analysis of normal and dysplastic ears and its implication on total auricular reconstruction", JOURNAL OF PLASTIC, RECONSTRUCTIVE AND AESTHETIC SURGERY, CHURCHILL LIVINGSTONE, GB, vol. 61, 18 April 2008 (2008-04-18), pages S21-S28, XP025795971, ISSN: 1748-6815, DOI: 10.1016/J.BJPS.2008.01.004 [retrieved on 2008-12-16]

## Description

### BACKGROUND OF THE INVENTION

Plastic and reconstructive surgery often entails the localization and clinical evaluation of a flap of skin and subcutaneous tissue which is supplied by isolated perforator vessels and that is potentially suitable for grafting in another part of the body Perforators pass from their source vessel to the skin surface, either through or between deep muscular tissues. Well-vascularised flaps are good candidates for grafts.

For example, abdominal donor-site flaps have become the standard for autologous breast reconstruction since the early 1980s. Within the abdomen, free fat options range from complete transverse rectus abdominis musculocutaneous (TRAM) flaps to isolated perforator flaps, such as the deep inferior epigastric artery (DIEA) perforator flap. Perforator flaps have allowed the transfer of the patient's own skin and fat in a reliable manner also in other areas of tissue reconstruction, with minimal donor-site morbidity Flaps that relied on a random pattern blood supply were soon supplanted by pedicled, axial patterned flaps that could reliably transfer great amounts of tissue. The advent of free tissue transfer allowed an even greater range of possibilities to appropriately match donor and recipient sites. The increased use of perforator flaps has escalated the need for a pre-operative familiarity of an individual's particular anatomical feature of the DIEA and its perforating branches, particularly given the significant variation in that anatomy of the vascular supply to the abdominal wall.

Localization and evaluation of perforators is a painstaking and time-consuming process. Pre-operative computed tomography angiographic (CTA) imaging is often performed to do the localization. Such an approach entails considerable expense and has the additional complication that the surgeon must mentally correlate the images from the previously acquired 3D modality with the current 2D view of the patient now lying on the operating table. The search for a more favorable imaging modality is thus continuing, with recent interest in the use of indocyanine green (ICG) fluorescence imaging, wherein blood circulation is assessed through the skin on the basis of a fluorescence signal. Fluorescence in ICG with an emission peak around 830 nm occurs as a result of excitation by radiation in the near-infrared spectral range. Excitation light with a wavelength around 800 nm can be produced, for example, by a diode laser, light emitting diodes (LED), or other conventional illumination sources, such as arc lamps, halogen lamps with a suitable bandpass filter. The skin is transparent to this wavelength.

State of the art ICG fluorescence imaging is for example described in:
HOLM ET AL: "Intraoperative evaluation of skin-flap viability using laser-induced fluorescence of indocyanine green",
BRITISH JOURNAL OF PLASTIC SURGERY, CHURCHILL LIVINGSTONE, GB,
vol. 55, no. 8, pages 635-644, XP005315304,
ISSN: 0007-1226, DOI: 10.1054/BJPS.2002.3969,
KRISHNAN K G ET AL: "The role of near-infrared angiography in the assessment of post-operative venous congestion in random pattern, pedicled island and free flaps",
BRITISH JOURNAL OF PLASTIC SURGERY, CHURCHILL LIVINGSTONE, GB,
vol. 58, no. 3, pages 330-338, XP004787587,
ISSN: 0007-1226, DOI: 10.10167J.BJPS.2004:10.003, and
US 2005/182434 A1 (DOCHERTY JOHN C [CA] ET AL) 18 August 2005 (2005-08-18).

ICG strongly binds to blood proteins and has previously been used for cardiac output measurement, hepatic function evaluation, and ophthalmic angiography, with few adverse reactions. Evaluation of ICG fluorescence signals can be used to locate perforators. Since the skin surface near a perforator generally accumulates more blood and at a faster rate than the surrounding tissue, once ICG is injected, perforators tend to fluoresce brighter and faster than the surrounding tissue. This rapid, high-intensity fluorescence enables visual localization of the perforator. Often, however, the surgeon is interested not merely in localization but also in evaluation and comparison to support good clinical decision making. The surgeon needs to decide which of several perforators the best graft candidates are. Here, simple visual observation while fluorescence rapidly accumulates and dissipates does not suffice. For example, the tendency of residual ICG from successive injections to accumulate in tissue and to gradually raise the background brightness with each injection further confounds easy visual discrimination of the best candidate perforators. In addition, ICG sometimes moves exceedingly slowly over several minutes making such on-the-fly analysis very challenging and subjective. A surgeon will make an assessment by raising the following questions:
1) How much ICG-bound blood is in the tissue?
2) How long does it stay in the tissue?
3) How quickly does it move through the tissue?
4) After the bolus is injected, in which order do anatomical areas light up?

These questions are difficult to answer on a subjective basis. Accordingly, there is a need for more advanced image processing and display methods to apply objective standards to localize and evaluate perforators.

### SUMMARY OF THE INVENTION

A method for preoperative identification of a perforator vessel for plastic and/or reconstructive surgery using ICG fluorescence angiography imaging is disclosed, which includes time-resolved image processing to highlight perforator locations and to enable visual discrimination among candidate perforators by various computed metrics. The surgeon is able to select and compare the results-of algorithms that analyze the time series and output the metrics according to at least one of the following processing acts:
Determine time-integrated fluorescence on a pixel-by-pixel basis.
Compute an average fluorescence by dividing the time-integrated fluorescence by the elapsed time.
Determine a rate of increase/wash-out in the fluorescence.
Determine the elapsed time to achieve peak fluorescence.

The various image processing steps process the image pixels independently and compute a unique numerical metric for each pixel in the input sequence observed across the entire time of the acquisition or a selected temporal sub-range. Each image output is thus a numerical array having the same dimensions, i.e. number and arrangement of pixels, as a frame in the input image sequence. Thus, the processed image can be displayed, for example, as a three-dimensional representation, for example a contour map, of the computed pixel values across the imaged area, or as a color-coded two-dimensional image or a relief map. Such image representations facilitate rapid comprehension of image features and comparison between regions on the images, in this case the location of the perforators under the skin.

These and other features and advantages of the present invention will become more readily appreciated from the detailed description of the invention that follows.

### BRIEF DESCRIPTION OF THE DRAWINGS

The following Figures depict certain illustrative embodiments in which like reference numerals refer to like elements. These depicted embodiments are to be understood as illustrative and not as limiting in any way.
FIG. 1 shows schematically a camera system for observing ICG fluorescence;
FIG. 2 shows an ICG fluorescence image of an area of skin, with the pixel values integrated over time;
FIG. 3 shows an ICG fluorescence image of an area of skein integrated over time, with the pixel values inversely weighted by elapsed time;
FIG. 4 shows an ICG fluorescence image of an area of skin, with the pixel values determined by the rate of increase of fluorescence;
FIG. 5 shows an ICG fluorescence image of an area of skin, with the pixel values determined by elapsed time to maximum fluorescence; and
FIG 6 shows an ICG fluorescence image of an area of skin, with the pixel values determined by peak fluorescence;
FIG. 7 shows an overlay of the fluorescence image processed with a variable contrast transfer function; and
FIG. 8 shows an overlay of the fluorescence image processed with another variable contrast transfer function.

### DETAILED DESCRIPTION

The disclosure is directed to preoperative determination of the location of perforator vessels in perforator flaps by a non-invasive method, before any incision is made.

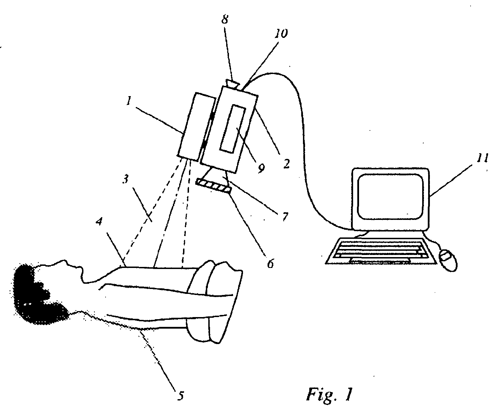

FIG. 1 shows schematically a device for a non-invasive, through the skin determination of tissue perfusion in operative in particular preoperative, applications by ICG fluorescence imaging. An infrared light source, for example, one or more diode lasers or LEDs, with a peak emission of about 780-800 nm for exciting fluorescence in ICG is located inside housing 1. The fluorescence signal is detected by a CCD camera 2 having adequate near-IR sensitivity; such cameras are commercially available from several vendors (Hitachi, Hamamatsu, etc.). The CCD camera 2 may have a viewfinder 8, but the image may also be viewed during the operation on an external monitor which may be part of an electronic image processing and evaluation system 11.

A light beam 3, which may be a divergent or a scanned beam, emerges from the housing 1 to illuminate an area of interest 4. i.e. the area where a flap with suitable perforator vessels is expected to be located. The area of interest may be about 10 cm x 10 cm, but may vary based on surgical requirements and the available illumination intensity and camera sensitivity.

A filter 6 is typically placed in front of the camera lens 7 to block excitation light from reaching the camera sensor, while allowing fluorescence light to pass through. The filter 6 may be an NIR long-wave pass filter (cut filter), which is only transparent to wavelengths greater than about 815 nm, or preferably a bandpass filter transmitting at peak wavelengths of between 830 and 845 nm and having a full width at half maximum (FWHM) transmission window of between about 10 nm and 25 nm, i.e. outside the excitation wavelength band. The camera 2 may also be designed to acquire a color image of the area of interest to allow real-time correlation between the fluorescence image and the color image.

In the present context the device illustrated in FIG. 1 is used to identify/ locate perforator vessels prior to surgery- this will assist the surgeon in selecting the best flap or flap zone for use during the reconstruction.

In other post-operative applications, the device can be used to:
Validate anastomotic patency and arterial and venous flow - this can potentially improve outcomes to eliminate flap failure which can be a result of poor arterial flow and inadequate perfusion as well as poor venous return resulting in congestion.
Visualize and confirm complete tissue perfusion, as micro-vascular perfusion to the entire flap and native tissue is critical to flap survival.

Perforator locations are visualized by image processing and presentation techniques to enable easy and objective visual discrimination among candidate perforators. ICG is injected and the entire ICG fluorescence perfusion and wash-out cycle is captured by the imaging device. After image acquisition, the entire sequence or some temporal sub-range of the images is processed by an image processing algorithm, which may be selected by the surgeon.

Processed results of the fluorescence measurements may be visualized, for example, as false color images or as a contour map, to enable rapid visual evaluation according to the applied algorithm metric. For example, the fluorescence intensity for each pixel may be rendered as a spectral color varying from blue ("cool" spots or low fluorescence-intensity or rate) to red ("hot" spots or high fluorescence- intensity or rate). Other spectral associations are easily accommodated. The output may be presented as a semitransparent overlay on the original anatomical images. This enables visual correlation of "hot" spots with the underlying anatomy. The meaning of "hot" spots varies with the algorithm employed, such as integrated intensity, weighted or unweighted, rate of increase or wash-out.

The user is given interactive control over the "hot" to "cool" color mapping and can vary it in real time to explore finer or coarser sub-ranges of the dynamic range of each algorithm's output metric. As the color window is widened, the hottest regions are highlighted first, followed by the cooler regions. This kind of adjustment can be made by changing the mapping of luminosity or contrast between the acquired pixels and the pixels in the displayed image. Such mapping functions may be included in standard imaging programs. This windowing process based on the currently employed metric aids in discriminating between perforators and enhances perception and improves understanding by the surgeon of the applied ICG dynamics.

The disclosure also supports the simultaneous display and evaluation of two sequences from two different locations on the patient's skin. This enables comparison of candidate flaps that are separated by a distance greater than the imaging system's field of view.

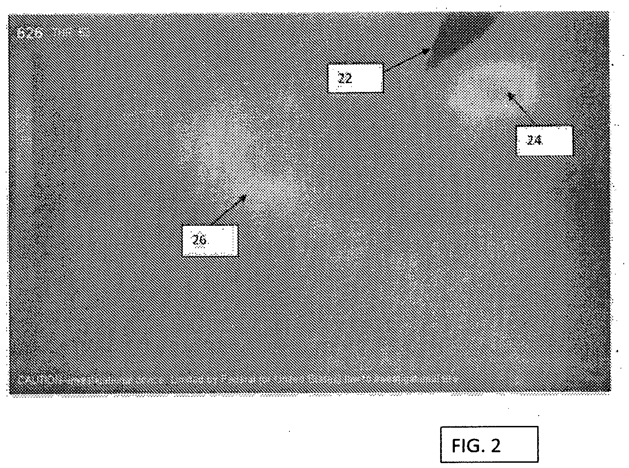

FIG. 2 shows an image of an area of a patient's skin where suitable perforator vessels are to be identified. Each pixel represents the time integral of fluorescence intensity over the exposure time for the image sequence. This mode is typically referred to as "integration mode" in image processing and many image processors offer this mode as a standard feature. In practice, the pixel intensities (collected charges in a CCD) acquired during each frame in the image sequence are added on a pixel-by-pixel basis, for example in the image processor, and divided by the number of frames, whereafter the sum may be normalized to a fixed dynamic range, for example, from 1 to 255 (8-bits). The notion is that brighter pixels in an image represent an area of the skin infused with a greater volume of blood carrying ICG over a preset period of time. In FIG. 2, the perforator vessel 24 exhibits the highest integrated fluorescence intensity, with another perforator vessel exhibiting weaker fluorescence intensity shown as 26.

Note that the transparency of the image has been set such that the physician's marker 22 is visible through the transparent color overlay of the ICG fluorescence image at the upper right of the screen.

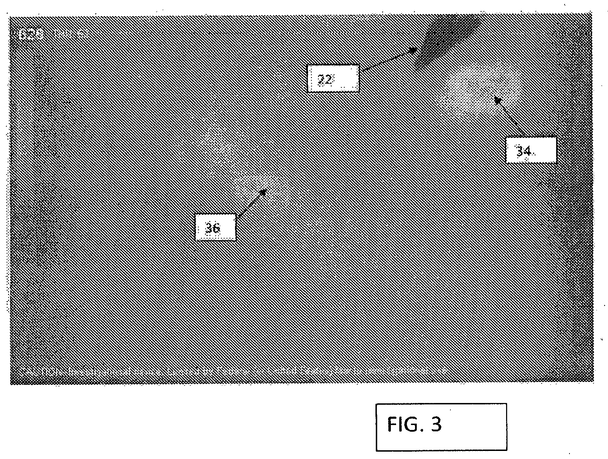

FIG. 3 shows an ICG fluorescence image of the same area of skin integrated over time, with the pixel values inversely weighted by elapsed time. This image processing algorithm is similar to the previously described integration, but instead of adding the measured intensities of each pixel directly, the measured intensity values are first divided by the elapsed time after start of the observation of ICG fluorescence, before being added. In this way, earlier fluorescence signals are given a greater significance that fluorescence signals acquired later. The "hottest" pixels are those pixels that in the sequence of image frames fluoresce earlier than other pixels which the ICG bolus reaches at a later time. The same perforator vessel 34 is identified as in FIG. 2, with another vessel 36 barely identifiable.

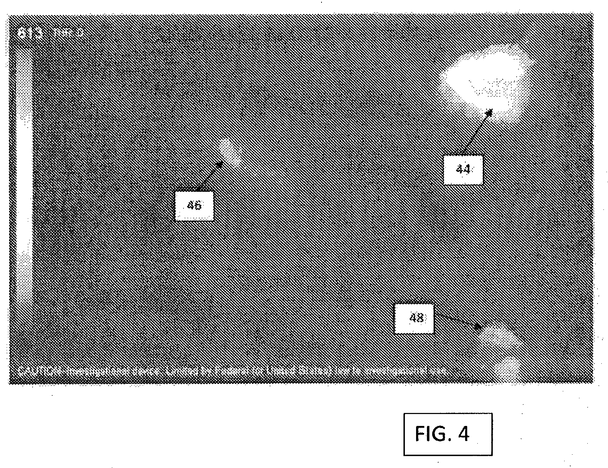

FIG. 4 shows an ICG fluorescence image, again of the same area of skin, with the pixel values in this image determined by the rate of increase of fluorescence intensity. In this image processing algorithm, a slope of the pixel intensity versus elapsed time is computed for each pixel in an image. For example, each pixel may have an assigned lowest intensity value (baseline) and an assigned highest intensity value (or another relatively high-intensity value). For each pixel, the time when the pixel intensity crosses the baseline and the time when the pixel intensity crosses the high-intensity value are noted. From this information, the image processing algorithm computes a rate of increase for each pixel in the image, with "hotter" pixels having a greater slope, i.e., they reach the high-intensity value faster than "cooler" pixels. This embodiment of the image processing algorithm thus highlights the speed at which the ICG bolus reaches the perforator vessels. The transparency has been turned off in FIG. 4, so that the surgeon's tool is not visible in the image.

The previously identified perforator vessel, shown here with the reference symbol 44, is much better defined, as are the vessel 46 (previously shown as 26 and 36) and another vessel 48.

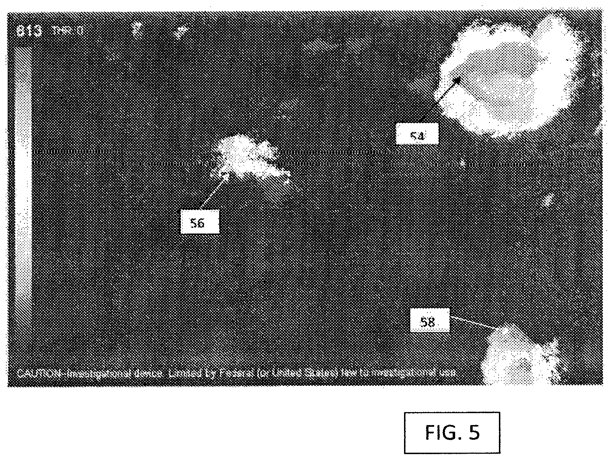

FIG. 5 shows an ICG fluorescence image of the same area of skin, with the pixel values determined by elapsed time to maximum fluorescence. Unlike FIG. 4, which displays the time rate of change, the image processing algorithm of FIG. 5 displays the time at which pixels reach their maximum intensity, with the "hotter" pixels reaching their respective peak fluorescence intensity sooner than cooler pixels. The algorithm thus highlights areas of the image in the order in which perforators reach their peak intensity. In this image, the previously identified perforator vessels 24, 34, 44 is again clearly distinguishable, as are the vessels 56 and 58 which correspond to the vessels 46 and 48 of FIG. 4.

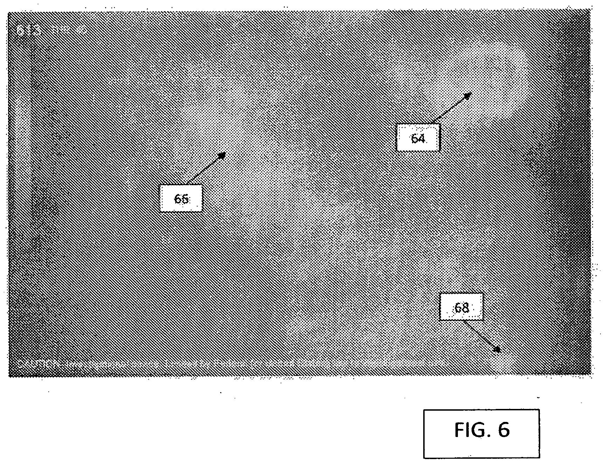

FIG. 6 shows an ICG fluorescence image of the same area of skin, with the pixel values determined by the peak fluorescence value at each pixel. Higher ("hot") fluorescence intensity values 64 may indicate a higher ICG concentration or may be caused by perforator vessels located closer to the skin surface, which reduces absorption of the excitation light /fluorescence response. The vessels 66, 68 which were clearly visible in FIGS. 4 and 5, are barely distinguishable from the background.

While the images shown, for example, in FIGS. 2 and 6 are rendered with a linear contrast transfer function providing a 1:1 mapping of pixel values processed with the various algorithms described above to the displayed pixel intensities, images can also be rendered (as contour maps or false color overlays) with a variable contrast transfer function to enhance the visual differences in the image. In addition, labels may be placed in the overlay images, hereinafter referred to as ACR (accumulated or time-integrated intensity ratio) labels, which facilitate a quantitative comparison between two or more regions of the anatomy.

Because absolute pixel values in the image change when the dynamic range and slope of the variable contrast transfer function is modified, the ACR labels allow the user to compare the relative perfusion in different image regions as measured by any of the selected overlay techniques (e.g. accumulated/ time-averaged intensity, et.).

The following approach is used to compute the ACR label values. For clarity, we assume that accumulated intensity is selected as overlay technique, although the same approach can be used with any of the available overlay techniques.
1) The accumulated intensity for all pixels for all images in the image sequence is computed over a time window.
2) The accumulated intensity is averaged over a region of the selected label (for example, a 5x5 pixel square matrix).
3) The averaged intensity is normalized to the maximum value of the accumulated intensity in the entire image.
4) The normalized average is scaled, with the maximum value of the transfer function representing 100%.

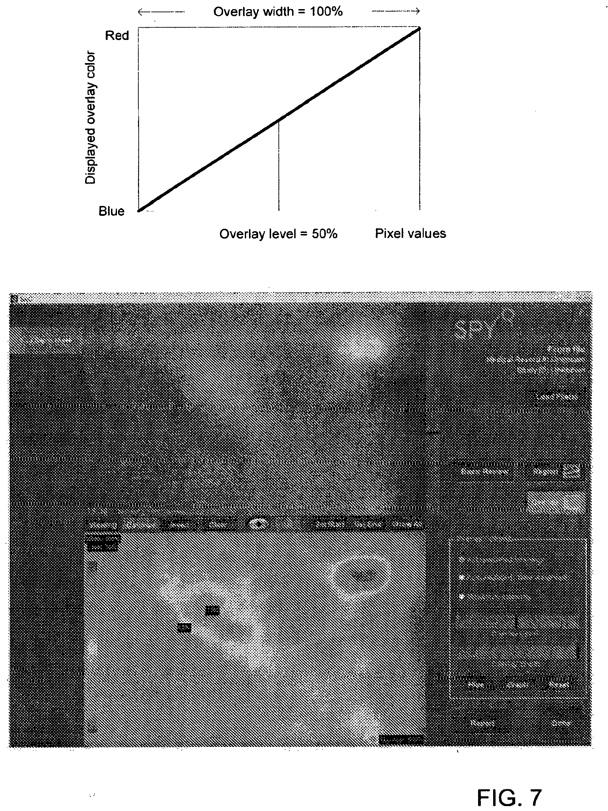

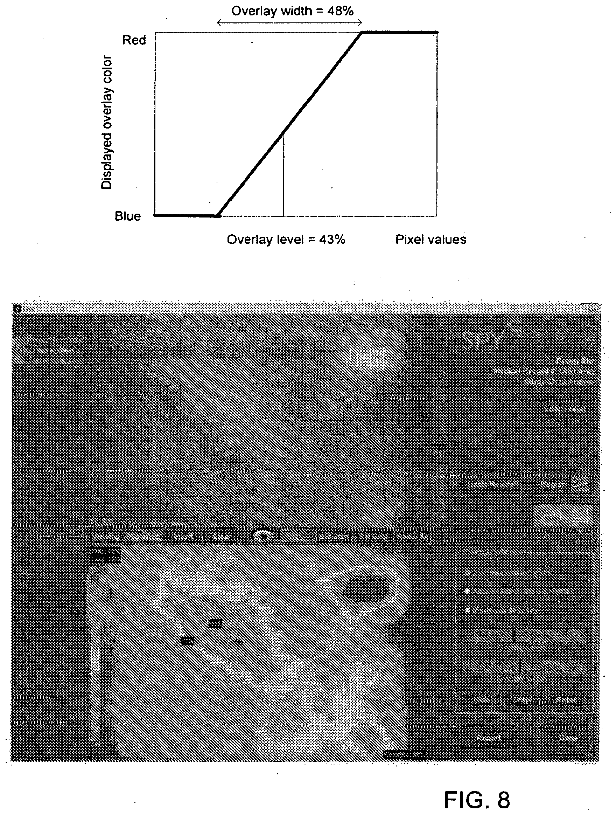

By following this approach, the relative ratio of two different ACR labels remains unchanged even though the slope of the transfer function is modified. FIGS. 7 and 8 show a fluorescence image from the image sequence that has been processed with one of the aforementioned algorithms (upper part of the gray-scale image) and the false-color overlay image rendering the accumulated intensity from the sequence in color (from blue for low values to red for high values) for two different contrast functions. The pixel values in FIG. 7 are processed with a first contrast transfer function, giving two regions with 52% and 72% intensity, respectively, corresponding to a ratio of 52/72 = .72 between the two labeled regions. The second overlay image in FIG 8 shows the same pixel values processed with a different contrast transfer function, with the intensity in the two regions now labeled 99% and 71%, respectively. However, their relative ratio remains essentially unchanged at 71/99 = 0.72.

The user can modify the transfer function so that a control region is labeled at 100%, wherein all other regions could then be compared to the control region.

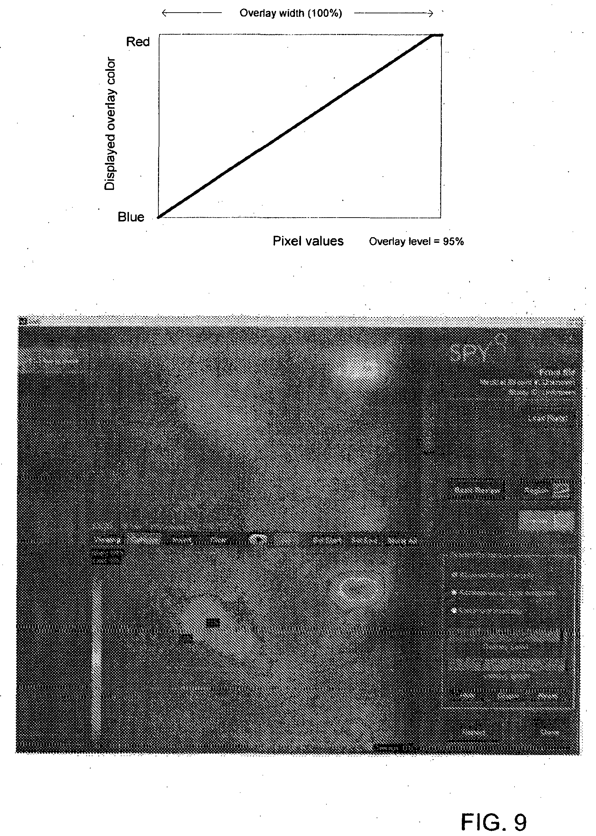

FIG. 9 shows that the overlay is transparent where accumulated intensity pixels have a value less than the point where the bottom of the transfer function ramp intersects the horizontal pixel value axis. Further, this demonstrates that in this example 12% of the image area (Coverage number in the lower right of the bottom window) has accumulated intensity greater than 52% of the maximum accumulated intensity. The illustration shows several regions bounded by their 52% contours.

The described embodiments detect a fluorescence signal emitted transcutaneously by ICG following excitation in the near-infrared spectral range. However, those of skill in the art will appreciate that other dyes which can be excited and emit fluorescence in a spectral range where tissue transmits light can also be used.

While the present disclosure has been described with reference to an example of arterial blood flow, i.e. supply of blood to the perforator vessel(s), the method may also detect graft failure due to venous congestion by quantifying and displaying the rate of change from peak intensity back down to the baseline. This will highlight venous return in the perfusion area.

While the disclosure is receptive to various modifications, and alternative forms, specific examples thereof have been shown in the drawings and are herein described in detail. It should be understood, however, that the invention is not limited to the particular forms or methods disclosed, but to the contrary, the invention is meant to cover all modifications, equivalents, and alternatives falling within the scope of the appended claims.

## Claims

1. A method for evaluating perfusion of perforator vessels, the method comprising the steps of
detecting a fluorescence response from the tissue following application of a bolus of indocyanine green (ICG) into the bloodstream of the subject;
acquiring a temporal sequence of images of the fluorescence response over a predetermined time;
processing the sequence of images by processing the image pixels independently and computing a unique numerical metric for each pixel in the input sequence observed across the entire time of the acquisition or a selected temporal subrange, to yield a time-integrated fluorescence intensity or a rate of increase of the fluorescence intensity for each pixel; and
displaying the time-integrated fluorescence intensity or the rate of increase of the fluorescence intensity as a color or black-and-white image,
wherein the method excludes the step of application of the bolus of ICG into the bloodstream.

2. The method of claim 1, further comprising applying a contrast transfer function to the time-integrated fluorescence intensity or the rate of increase of the fluorescence intensity.

3. The method of claim 2, wherein the contrast transfer function represents a linear or nonlinear function which transforms the time-integrated fluorescence intensity or the rate of increase of the fluorescence intensity into an overlay image representing different perfusion characteristics in different colors.

4. The method of claim 2 or 3, wherein the contrast transfer function is a non-linear function with regions of different slope, and wherein the slopes and a transition between the different slopes is selectable during a procedure to evaluate the tissue perfusion.

5. The method of claim 3 or 4 when dependent on claim 3, wherein the time-integrated fluorescence intensity or the rate of increase of the fluorescence intensity are displayed as numerical values in the overlay image.

6. The method of one of claims 2 to 5 further comprising computing a time-integrated fluorescence intensity ratio for different image regions of an anatomical feature.

7. The method of claim 6, wherein computing the time-integrated fluorescence intensity ratio comprises the following steps:
computing the time-integrated fluorescence intensity for all pixels,
averaging the time-integrated fluorescence intensity over a predefined region in the image,
normalizing the averaged time-integrated fluorescence intensity to a maximum value of the time-integrated fluorescence intensity in the entire image; and
scaling the normalized time-integrated fluorescence intensity with a maximum value of the contrast transfer function.

8. An apparatus for evaluating perfusion of perforator vessels, the apparatus comprising:
means (2) for detecting a fluorescence response from the tissue following application of a bolus of ICG into the bloodstream of the subject,
means (11) for acquiring a temporal sequence of images of the fluorescence response over a predetermined time,
means (11) for processing the sequence of images by processing the image pixels independently and computing a unique numerical metric for each pixel in the input sequence observed across the entire time of the acquisition or a selected temporal subrange, to yield a time-integrated fluorescence intensity or a rate of increase of the fluorescence intensity for each pixel; and
means (11) for displaying the time-integrated fluorescence intensity or rate of increase of the fluorescence intensity as a color or black-and-white image.

9. Indocyanine green (ICG), for use in a method for evaluating perfusion of perforator vessels, the method comprising the steps of:
Application of a bolus of ICG into the bloodstream of the subject;
detecting a fluorescence response from the tissue following said application of the bolus of ICG into the bloodstream of the subject;
acquiring a temporal sequence of images of the fluorescence response over a predetermined time;
processing the sequence of images by processing the image pixels independently and computing a unique numerical metric for each pixel in the input sequence observed across the entire time of the acquisition or a selected temporal subrange, to yield a time-integrated fluorescence intensity or rate of increase of the fluorescence intensity for each pixel; and
displaying the time-integrated fluorescence intensity or the rate of increase of the fluorescence intensity as a color or black-and-white image.

10. ICG of claim 9, wherein the method further comprises applying a contrast transfer function to the time-integrated fluorescence intensity or the rate of increase of the fluorescence intensity.

11. ICG of claim 10, wherein the contrast transfer function represents a linear or a nonlinear function which transforms the time-integrated fluorescence intensity or the rate of increase of the fluorescence intensity into an overlay image representing different perfusion characteristics in different colors.

12. ICG of claim 10 or claim 11, wherein the contrast transfer function is a nonlinear function with regions of different slope, and wherein the slopes and a transition between the different slopes is selectable during a procedure to evaluate the tissue perfusion.

13. ICG of any one of claims 11 or 12 when dependent on claim 11, wherein the method further comprises displaying the time-integrated fluorescence intensity or the rate of increase of the fluorescence intensity as numeral values in the overlay image.

14. ICG of any one of claims 10 to 13, wherein the method further comprises computing a time-integrated fluorescence intensity ratio for different image regions of an anatomical feature.

15. ICG of claim 14, wherein the time-integrated fluorescence intensity ratio is computed with the following steps:
computing the time-integrated fluorescence intensity for all pixels;
averaging the time-integrated fluorescence intensity over a predefined region in the image;
normalizing the averaged time-integrated fluorescence intensity to a maximum value of the time-integrated fluorescence intensity in the entire image; and
scaling the normalized time-integrated fluorescence intensity with a maximum value of the contrast transfer function.

## Patentansprüche

1. Verfahren zur Bewertung der Perfusion von Perforansgefäßen, das Verfahren umfassend folgende Schritte:
Erkennen einer Fluoreszenzreaktion von dem Gewebe nach der Anwendung eines Bolus von Indocyaningrün (ICG) im Blutstrom des Subjekts;
Erfassen einer temporalen Sequenz von Bildern der Fluoreszenzreaktion über eine vorbestimmte Zeit;
Verarbeiten der Sequenz von Bildern durch unabhängige Verarbeitung der Bildpixel und Berechnung einer eindeutigen numerischen Metrik für jedes Pixel in der eingegebenen Sequenz, beobachtet über die gesamte Zeit der Erfassung oder einen ausgewählten temporalen Teilbereich, um eine zeitintegrierte Fluoreszenzintensität oder eine Zunahmerate der Fluoreszenzintensität für jedes Pixel zu erhalten; und
Anzeigen der zeitintegrierten Fluoreszenzintegrität oder der Zunahmerate der Fluoreszenzintensität als ein Farb- oder Schwarz-Weiß-Bild,
wobei das Verfahren den Schritt der Anwendung des Bolus von ICG in dem Blutstrom ausschließt.

2. Verfahren nach Anspruch 1, ferner umfassend die Anwendung einer Kontrastübertragungsfunktion auf die zeitintegrierte Fluoreszenzintensität oder die Zunahmerate der Fluoreszenzintensität.

3. Verfahren nach Anspruch 2, wobei die Kontrastübertragungsfunktion eine lineare oder nicht lineare Funktion darstellt, die die zeitintegrierte Fluoreszenzintensität oder die Zunahmerate der Fluoreszenzintensität in ein Überlagerungsbild umwandelt, das verschiedene Perfusionsmerkmale in verschiedenen Farben darstellt.

4. Verfahren nach Anspruch 2 oder 3, wobei die Kontrastübertragungsfunktion eine nicht lineare Funktion mit Bereichen von unterschiedlicher Neigung ist und wobei die Neigungen und ein Übergang zwischen den verschiedenen Neigungen in einem Verfahren zur Bewertung der Gewebeperfusion wählbar ist.

5. Verfahren nach Anspruch 3 oder 4, wenn abhängig von Anspruch 3, wobei die zeitintegrierte Fluoreszenzintensität oder die Zunahmerate der Fluoreszenzintensität als Zahlenwerte in dem Überlagerungsbild angezeigt werden.

6. Verfahren nach einem der Ansprüche 2 bis 5, ferner umfassend die Berechnung eines zeitintegrierten Fluoreszenzintensitätsverhältnisses für verschiedene Bildbereiche eines anatomischen Merkmals.

7. Verfahren nach Anspruch 6, wobei die Berechnung des zeitintegrierten Fluoreszenzintensitätsverhältnisses folgende Schritte umfasst:
Berechnen der zeitintegrierten Fluoreszenzintensität für alle Pixel,
Bilden des Mittelwerts der zeitintegrierten Fluoreszenzintensität über einen vordefinierten Bereich in dem Bild,
Normalisieren der gemittelten zeitintegrierten Fluoreszenzintensität auf einen Höchstwert der zeitintegrierten Fluoreszenzintensität in dem Gesamtbild; und
Skalieren der normalisierten zeitintegrierten Fluoreszenzintensität mit einem Höchstwert der Kontrastübertragungsfunktion.

8. Vorrichtung zur Bewertung der Perfusion von Perforansgefäßen, die Vorrichtung umfassend:
Mittel (2) zum Erkennen einer Fluoreszenzreaktion von dem Gewebe nach der Anwendung eines Bolus von ICG im Blutstrom des Subjekts,
Mittel (11) zum Erfassen einer temporalen Sequenz von Bildern der Fluoreszenzreaktion über eine vorbestimmte Zeit,
Mittel (11) zur Verarbeitung der Sequenz von Bildern durch unabhängige Verarbeitung der Bildpixel und Berechnung einer eindeutigen numerischen Metrik für jedes Pixel in der eingegebenen Sequenz, beobachtet über die gesamte Zeit der Erfassung oder einen ausgewählten temporalen Teilbereich, um eine zeitintegrierte Fluoreszenzintensität oder eine Zunahmerate der Fluoreszenzintensität für jedes Pixel zu erhalten; und
Mittel (11) zum Anzeigen der zeitintegrierte Fluoreszenzintensität oder der Zunahmerate der Fluoreszenzintensität als ein Farb- oder Schwarz-Weiß-Bild.

9. Indocyaningrün (ICG) zur Verwendung in einem Verfahren zur Bewertung der Perfusion von Perfusansgefäßen, das Verfahren umfassend folgende Schritte:
Anwenden eines Bolus von ICG im Blutstrom des Subjekts;
Erkennen einer Fluoreszenzreaktion von dem Gewebe nach der Anwendung des Bolus von ICG im Blutstrom des Subjekts;
Erfassen einer temporalen Sequenz von Bildern der Fluoreszenzreaktion über eine vorbestimmte Zeit;
Verarbeiten der Sequenz von Bildern durch unabhängige Verarbeitung der Bildpixel und Berechnung einer eindeutigen numerischen Metrik für jedes Pixel in der eingegebenen Sequenz, beobachtet über die gesamte Zeit der Erfassung oder einen ausgewählten temporalen Teilbereich, um eine zeitintegrierte Fluoreszenzintensität oder eine Zunahmerate der Fluoreszenzintensität für jedes Pixel zu erhalten; und
Anzeigen der zeitintegrierten Fluoreszenzintensität oder der Zunahmerate der Fluoreszenzintensität als ein Farb- oder Schwarz-Weiß-Bild.

10. ICG nach Anspruch 9, wobei das Verfahren ferner die Anwendung einer Kontrastübertragungsfunktion auf die zeitintegrierte Fluoreszenzintensität oder die Zunahmerate der Fluoreszenzintensität umfasst.

11. ICG nach Anspruch 10, wobei die Kontrastübertragungsfunktion eine lineare oder eine nicht lineare Funktion darstellt, die die zeitintegrierte Fluoreszenzintensität oder die Zunahmerate der Fluoreszenzintensität in ein Überlagerungsbild umwandelt, das verschiedene Perfusionsmerkmale in verschiedenen Farben darstellt.

12. ICG nach Anspruch 10 oder Anspruch 11, wobei die Kontrastübertragungsfunktion eine nicht lineare Funktion mit Bereichen von verschiedener Neigung ist und wobei die Neigungen und ein Übergang zwischen den verschiedenen Neigungen in einem Verfahren zur Bewertung der Gewebeperfusion wählbar ist.

13. ICG nach einem der Ansprüche 11 oder 12, wenn abhängig von Anspruch 11, wobei das Verfahren ferner die Anzeige der zeitintegrierten Fluoreszenzintensität oder der Zunahmerate der Fluoreszenzintensität als Zahlenwerte in dem Überlagerungsbild umfasst.

14. ICG nach einem der Ansprüche 10 bis 13, wobei das Verfahren ferner die Berechnung eines zeitintegrierten Fluoreszenzintensitätsverhältnisses für verschiedene Bildbereiche eines anatomischen Merkmals umfasst.

15. ICG nach Anspruch 14, wobei das zeitintegrierte Fluoreszenzintensitätsverhältnis in folgenden Schritten berechnet wird:
Berechnung der zeitintegrierten Fluoreszenzintensität für alle Pixel;
Bilden eines Mittelwerts der zeitintegrierten Fluoreszenzintensität über einen vordefinierten Bereich in dem Bild;
Normalisieren der gemittelten zeitintegrierten Fluoreszenzintensität auf einen Höchstwert der zeitintegrierten Fluoreszenzintensität in dem Gesamtbild; und
Skalieren der normalisierten zeitintegrierten Fluoreszenzintensität mit einem Höchstwert der Kontrastübertragungsfunktion.

## Revendications

1. Procédé d'évaluation de perfusion de vaisseaux perforants, le procédé comprenant les étapes consistant à :
détecter une réponse de fluorescence du tissu après l'application d'un bol de vert d'indocyanine (ICG) au flux sanguin du sujet ;
acquérir une séquence temporelle d'images de la réponse de fluorescence sur une durée prédéterminée ;
traiter la séquence d'images en traitant les pixels d'image de manière indépendante, et en calculant une valeur numérique unique pour chaque pixel de la séquence d'entrée observée pendant l'intégralité de la durée d'acquisition ou une sous-plage temporelle sélectionnée, afin d'obtenir une intensité de fluorescence intégrée dans le temps ou une vitesse d'augmentation de l'intensité de fluorescence pour chaque pixel ; et
afficher l'intensité de fluorescence intégrée dans le temps ou la vitesse d'augmentation de l'intensité de fluorescence sous la forme d'une image en couleur ou en noir et blanc,
dans lequel le procédé exclut l'étape d'application du bol de vert d'indocyanine au flux sanguin.

2. Procédé selon la revendication 1, comprenant en outre l'application d'une fonction de transfert de contraste à l'intensité de fluorescence intégrée dans le temps ou à la vitesse d'augmentation de l'intensité de fluorescence.

3. Procédé selon la revendication 2, dans lequel la fonction de transfert de contraste représente une fonction linéaire ou non-linéaire qui transforme l'intensité de fluorescence intégrée dans le temps ou la vitesse d'augmentation de l'intensité de fluorescence en une image de superposition représentant différentes caractéristiques de perfusion dans différentes couleurs.

4. Procédé selon la revendication 2 ou 3, dans lequel la fonction de transfert de contraste est une fonction non-linéaire avec des zones à pentes différentes, et dans lequel les pentes et un passage entre les différentes pentes peuvent être sélectionnés pendant une procédure d'évaluation de la perfusion d'un tissu.

5. Procédé selon la revendication 3 ou 4, lorsqu'elle dépend de la revendication 3, dans lequel l'intensité de fluorescence intégrée dans le temps ou la vitesse d'augmentation de l'intensité de fluorescence est affichée sous forme de valeur numérique sur l'image de superposition.

6. Procédé selon l'une des revendications 2 à 5, comprenant en outre le calcul d'un rapport d'intensité de fluorescence intégrée dans le temps pour différentes zones d'image d'une caractéristique anatomique.

7. Procédé selon la revendication 6, dans lequel le calcul du rapport d'intensité de fluorescence intégrée dans le temps comprend les étapes suivantes :
le calcul de l'intensité de fluorescence intégrée dans le temps pour l'ensemble des pixels,
le moyennage de l'intensité de fluorescence intégrée dans le temps sur une zone prédéfinie de l'image,
la normalisation de l'intensité de fluorescence intégrée dans le temps et moyennée par rapport à une valeur maximale de l'intensité de fluorescence intégrée dans le temps sur l'image entière ; et
la mise à l'échelle de l'intensité de fluorescence intégrée dans le temps et normalisée avec une valeur maximale de la fonction de transfert de contraste.

8. Appareil d'évaluation de la perfusion de vaisseaux perforants, l'appareil comprenant :
un moyen (2) destiné à détecter une réponse de fluorescence du tissu après l'application d'un bol de vert d'indocyanine au flux sanguin du sujet,
un moyen (11) destiné à acquérir une séquence temporelle d'images de la réponse de fluorescence pendant une durée prédéterminée,
un moyen (11) destiné à traiter la séquence d'images en traitant les pixels d'image de manière indépendante et en calculant une valeur numérique unique pour chaque pixel de la séquence d'entrée observée pendant l'intégralité de la durée d'acquisition ou une sous- plage temporelle sélectionnée, afin d'obtenir une intensité de fluorescence intégrée dans le temps ou une vitesse d'augmentation de l'intensité de fluorescence pour chaque pixel ; et
un moyen (11) destiné à afficher l'intensité de fluorescence intégrée dans le temps ou la vitesse d'augmentation de l'intensité de fluorescence sous la forme d'une image en couleur ou en noir et blanc.

9. Vert d'indocyanine (ICG) destiné à être utilisé dans un procédé d'évaluation de la perfusion de vaisseaux perforants, le procédé comprenant les étapes consistant à :
appliquer un bol de vert d'indocyanine au flux sanguin du sujet;
détecter une réponse de fluorescence du tissu après ladite application du bol de vert d'indocyanine au flux sanguin du sujet ;
acquérir une séquence temporelle d'images de la réponse de fluorescence pendant une durée prédéterminée ;
traiter la séquence d'images en traitant les pixels d'image de manière indépendante et en calculant une valeur numérique unique pour chaque pixel de la séquence d'entrée observée pendant l'intégralité de la durée d'acquisition ou une sous-plage temporelle sélectionnée, afin d'obtenir une intensité de fluorescence intégrée dans le temps ou une vitesse d'augmentation de l'intensité de fluorescence pour chaque pixel ; et
afficher l'intensité de fluorescence intégrée dans le temps ou la vitesse d'augmentation de l'intensité de fluorescence sous la forme d'une image en couleur ou en noir et blanc.

10. Vert d'indocyanine (ICG) selon la revendication 9, dans lequel le procédé comprend en outre l'application d'une fonction de transfert de contraste à l'intensité de fluorescence intégrée dans le temps ou à la vitesse d'augmentation de l'intensité de fluorescence.

11. Vert d'indocyanine (ICG) selon la revendication 10, dans lequel la fonction de transfert de contraste représente une fonction linéaire ou non-linéaire qui transforme l'intensité de fluorescence intégrée dans le temps ou la vitesse d'augmentation de l'intensité de fluorescence en une image de superposition représentant différentes caractéristiques de perfusion dans différentes couleurs.

12. Vert d'indocyanine (ICG) selon la revendication 10 ou 11, dans lequel la fonction de transfert de contraste est une fonction non-linéaire avec des zones à pentes différentes, et dans lequel les pentes et un passage entre les différentes pentes peuvent être sélectionnés pendant une procédure d'évaluation de la perfusion d'un tissu.

13. Vert d'indocyanine (ICG) selon l'une quelconque des revendications 11 ou 12, lorsqu'elles dépendent de la revendication 11, dans lequel le procédé comprend en outre l'affichage de l'intensité de fluorescence intégrée dans le temps ou de la vitesse d'augmentation de l'intensité de fluorescence sous la forme de valeurs numériques sur l'image de superposition.

14. Vert d'indocyanine (ICG) selon l'une quelconque des revendications 10 à 13, dans lequel le procédé comprend en outre le calcul d'un rapport d'intensité de fluorescence intégrée dans le temps pour différentes zones d'image d'une caractéristique anatomique.

15. Vert d'indocyanine (ICG) selon la revendication 14, dans lequel le rapport d'intensité de fluorescence intégrée dans le temps est calculé à l'aide des étapes suivantes :
le calcul de l'intensité de fluorescence intégrée dans le temps pour l'ensemble des pixels,
le moyennage de l'intensité de fluorescence intégrée dans le temps sur une zone prédéfinie de l'image ;
la normalisation de l'intensité de fluorescence intégrée dans le temps et moyennée par rapport à une valeur maximale de l'intensité de fluorescence intégrée dans le temps sur l'image entière ; et
la mise à l'échelle de l'intensité de fluorescence intégrée dans le temps et normalisée avec une valeur maximale de la fonction de transfert de contraste.
